(19) Europäisches Patentamt
European Patent Office
Office européen des brevets

(11) Veröffentlichungsnummer: **0 001 623**
**B1**

(12) # EUROPÄISCHE PATENTSCHRIFT

(21) Anmeldenummer: **78101143.2**

(22) Anmeldetag: **14.10.78**

(51) Int. Cl.³: **C 07 C 161/02,**
A 01 N 31/04, //
C 07 C 153/07

(54) **Aryl-thiocarbonsäure-thiocyanmethylester, ihre Herstellung und ihre Verwendung als Pestizide, sowie sie enthaltende Mittel**

(30) Priorität: **26.10.77 DE 2747825**

(43) Veröffentlichungstag der Anmeldung:
**02.05.79 Patentblatt 79/09**

(45) Bekanntmachung des Hinweises auf die Patenterteilung:
**03.09.80 Patentblatt 80/18**

(84) Benannte Vertragsstaaten:
**BE DE FR GB NL SE**

(56) Entgegenhaltungen:
**US - A - 3 095 437**
**US - A - 3 845 082**

**Archiv Der Pharmazie, Band 294, 1961 Weinheim, H. Böhme u.a. "Über Acetylmercaptomethyl-rhodanid, -senföl und -cyanid" Seite 475 bis 478**

**Archiv Der Pharmazie, Band 300, 1967, Weinheim, H. Böhme u.a. "Über Acetoxymethylrhodanid und Alkoxymethylsenföle" Seite 326 bis 329**

**Chemical Abstracts vol. 80, no. 20, 18 March 1974, Columbus Ohio, USA, Takahashi u.a. "Thiocyamate fungicides" Spalte 1, Abstract Nr. 56 457y**

(73) Patentinhaber: **Bayer AG**
**Zentralbereich Patente, Marken und Lizenzen**
**D - 5090 Leverkusen 1, Bayerwerk (DE)**

(72) Erfinder: **Oeckl, Siegfried, Dr.**
**Andreas-Gryphius-Strasse 9**
**D - 5000 Köln 80 (DE)**
**Genth, Hermann, Dr.**
**Am Heckerhof 60**
**D - 4150 Krefeld 1 (DE)**
**Paulus, Wilfried, Dr.**
**Deswatinesstrasse 90**
**D - 4150 Krefeld 1 (DE)**
**Rother, Heinz-Joachim, Dr.**
**Breslauer Strasse 31**
**D - 4150 Krefeld 1 (DE)**
**Stendel, Wilhelm Dr.**
**In den Birken 55**
**D - 5600 Wuppertal 1 (DE)**
**Brandes, Wilhelm, Dr.**
**Eichendorffstrasse 3**
**D - 5653 Leichlingen 1 (DE)**
**Kraus, Peter, Dr.**
**Duesseldorfer Strasse 43**
**D - 5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 623 B1

Aryl-thiocarbonsäure-thiocyanmethylester, ihre Herstellung und ihre Verwendung als Pestizide sowie sie enthaltende Mittel

Aus der US 3 095 437 sind $\alpha$-Thiocyanalkylester aromatischer Carbonsäuren bekannt, die eine mikrobizide Wirkung haben.

Es wurden neue Aryl-thiocarbonsäure-thiocyanmethylester der Formel

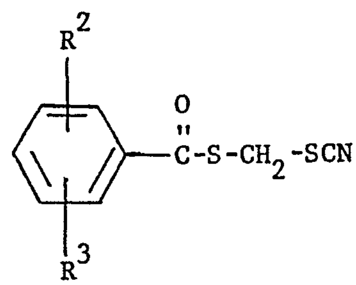

I

worin

$R^2$ und $R^3$ gleich oder verschieden sind und Wasserstoff, Halogen, $C_1$ bis $C_6$ Alkyl, durch Halogen substituiertes $C_1$ bis $C_6$ Alkyl, $C_1$ bis $C_6$ Alkoxy oder Nitro bedeutet,

gefunden.

Insbesondere werden Aryl-thiocarbonsäure-thiocyanmethylester der Formel

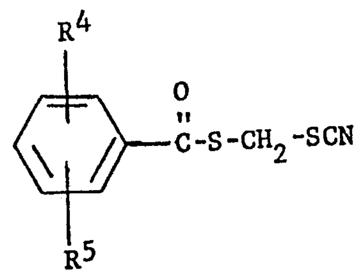

II

worin

$R^4$ und $R^5$ gleich oder verschieden sind und Wasserstoff, Chlor, Methyl, Ethyl, Trifluormethyl, Trichlormethyl, Methoxy oder Ethoxy bedeuten, bevorzugt.

Beispielsweise seien die folgenden Aryl-thiocarbonsäure-thiocyanmethylester genannt:

Thiobenzoesäure-thiocyanmethylester, 4-Chlor-thiobenzoesäure-thiocyanmethylester, 2,5-Dichlor-thiobenzoesäure-thiocyanmethylester, 4-Methylthiobenzoesäure-thiocyanmethylester, 2-Chlor-thiobenzoesäure-thiocyanmethylester, 3-Methyl-thiobenzoesäure-thiocyanmethylester, 3-Trifluormethyl-thiobenzoesäure-thiocyanmethylester und 2,4-Dibromthiobenzoesäure-thiocyanmethylester.

Außerdem wurde ein Verfahren zur Herstellung von Arylthiocarbonsäure-thiocyanmethylester gefunden, bei dem man Arylthiocarbonsäuren der Formel

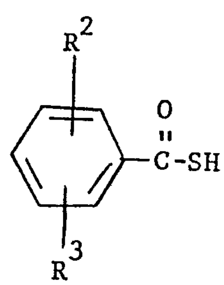

III

worin

$R^2$ und $R^3$ die obengenannte Bedeutung haben, in Gegenwart eines Säurebinders gegebenenfalls in Gegenwart eines Lösungsmittels mit einer Verbindung der Formel

$$X—CH_2—R^6 \qquad IV$$

worin

X für Halogen und

$R^6$ für Halogen oder die Thiocyangruppe steht, und dann gegebenenfalls mit Thiocyanat umsetzt.

Halogene ($R^2$, $R^3$, $R^6$ und X) können Fluor, Chlor, Brom und Jod, bevorzugt Chlor, sein.

Alkyl ($R^2$ und $R^3$) kann ein geradkettiger oder verzweigter Kohlenwasserstoffrest mit 1 bis 6 Kohlenstoffatomen sein; beispielsweise sei Methyl, Ethyl, Propyl, Isopropyl, Butyl, Isobutyl, Pentyl, Isopentyl, Hexyl und Isohexyl, bevorzugt Methyl und Ethyl, genannt.

Halogenalkyl ($R^2$ und $R^3$) kann ein geradkettiger oder verzweigter Kohlenwasserstoffrest mit 1 bis 6 Kohlenstoffatomen sein, der durch Fluor, Chlor, Brom und Jod, bevorzugt Fluor und Chlor, substituiert ist; beispielsweise seien genannt: Trifluormethyl, Trichlormethyl, Tribrommethyl, Pentachlorethyl, 1,1,1-Trichlorethyl und 1,1-Dichlor-1-fluor-ethyl.

Alkoxy ($R^2$ und $R^3$) kann einen geradkettiger oder verzweigten aliphatischen Kohlenwasserstoffrest mit 1 bis 6 Kohlenstoffatomen enthalten; beispielsweise seien Methoxy, Ethoxy, Propoxy, Isopropoxy, Butoxy, Isobutoxy, Pentoxy, Isopentoxy, Hexoxy und Isohexoxy genannt.

Insbesondere werden für das erfindungsgemäße Verfahren Arylthiocarbonsäuren der Formel

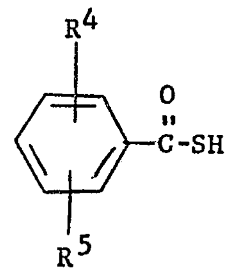

V

worin

$R^4$ und $R^5$ die obengenannte Bedeutung haben, bevorzugt.

Arylthiocarbonsäuren sind bekannt (Beilstein Bd. 9, 419 I, 169; Org. Synth. *37* 101) und können beispielsweise durch Umsetzung eines gegebenenfalls substituierten Benzoylchlorids mit Natriumhydrogensulfid hergestellt werden.

Beispielsweise seien die folgenden Arylthiocarbonsäuren genannt:

Thiobenzoesäure, 4-Chlor-Thiobenzoesäure, 2,5-Dichlor-thiobenzoesäure, 4-Methyl-thiobenzoesäure, 2-Chlor-thiobenzoesäure, 3-Ethyl-thiobenzoesäure, 3-Trifluormethyl-thiobenzoesäure und 2,4-Dibrom-thiobenzoesäure.

Als Säurebinder für das erfindungsgemäße Verfahren kommen organische oder anorganische basische Verbindungen wie tertiäre Amine, Metallalkoholate, Metallsalze von Carbonsäuren, Metallcarbonate und Hydroxide in Frage. Als Metalle seien die Alkali- und Erdalkalimetalle, aber auch Ammonium genannt. Es ist auch möglich, Säurebindersalze der Arylthiocarbonsäuren selbst einzusetzen. Beispielsweise seien genannt: Aryl-thiocarbonsäure-triäthylammoniumsalz, Aryl-thiocarbonsäure-dimethyl-benzylammoniumsalz, Arylthiocarbonsäure-pyridiniumsalze, Aryl-thiocarbonsäurenatrium-, Aryl-thiocarbonsäure-kalium-salz. Bevorzugte Säurebinder sind Natrium- und Kaliummethylat, Natrium- und Kaliumhydroxid, Natrium- und Kaliumcarbonat, Dimethylbenzylamin, Triäthylamin und Pyridin. Bevorzugte Säurebindersalze sind die Natrium-, Kalium- und Dimethylbenzylammoniumsalze der Thiobenzoesäure, 4'-Chor-thiobenzoesäure, 2,5-Dichlor-thiobenzoesäure, 4-Methyl-thiobenzoesäure, 2-Chlor-thiobenzoesäure, 3-Äthyl-thiobenzoesäure, 3-Trifluormethyl-thiobenzoesäure und 2,4-Dibrom-thiobenzoesäure.

Das erfindungsgemäße Verfahren kann ohne, vorzugsweise jedoch in Gegenwart eines Lösungsmittels durchgeführt werden. Als Lösungsmittel kommen alle gegenüber den Reaktionskomponenten inerten Lösungsmittel, wie beispielsweise Alkohole, Äther, Kohlenwasserstoffe, Halogenkohlenwasserstoffe, wie Äthanol, Methanol, Dioxan, Toluol, Chlorbenzol oder eine Reaktionskomponente, wie das Halogenmethylthiocyanat, in Frage. Es ist auch möglich, in Gegenwart von Wasser in einem Zweiphasensystem zu arbeiten.

In einer bevorzugten Ausführungsform des erfindungsgemäßen Verfahrens kann man die Arylthiocarbonsäure mit einem Halogenmethyl-thiocyanat umsetzen. Die Umsetzung kann durch die folgende Reaktionsgleichung erläutert werden:

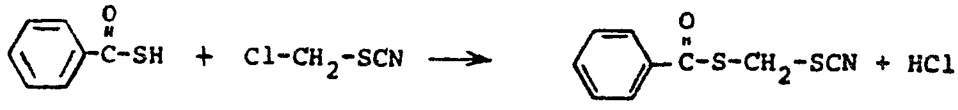

Als Halogenmethylthiocyanat wird für das erfindungsgemäße Verfahren insbesondere das Chlormethylthiocyanat bevorzugt.

Die Herstellung der Halogenmethylthiocyanate ist bekannt (Beilstein *3*, II, 124) und kann beispielsweise durch Umsetzung von Dihalogenmethan mit einem Mol Alkalithiocyanat hergestellt werden.

Im allgemeinen wird das erfindungsgemäße Verfahren mit äquivalenten Mengen der Reaktionspartner durchgeführt; es kann aber auch günstig sein, ein Überschuß der einen oder anderen Komponente einzusetzen. Die bevorzugte Ausführungsform des erfindungsgemäßen Verfahrens wird im allgemeinen im Temperaturbereich von —20 bis +100°C, vorzugsweise von —10° bis +50°C, durchgeführt.

Nach der Umsetzung erfolgt die Aufarbeitung üblicherweise durch Filtration, Verdampfen des Lösungsmittels und gegebenenfalls durch Waschen oder Umkristallisieren des Rückstands.

In einer anderen bevorzugten Ausführungsform des erfindungsgemäßen Verfahrens kann die Herstellung der erfindungsgemäßen Aryl-thiocarbonsäure-thiocyanmethylester in einer ersten Reaktionsstufe durch Umsetzung der Arylthio-carbonsäuren mit einem Dihalogenmethan zu einem Aryl-thiocarbonsäurehalogenmethylester, der in einer zweiten Reaktionsstufe mit einem Thiocyanat umgesetzt wird, erfolgen.

Die Umsetzung kann durch die folgenden Reaktionsgleichungen erläutert werden:

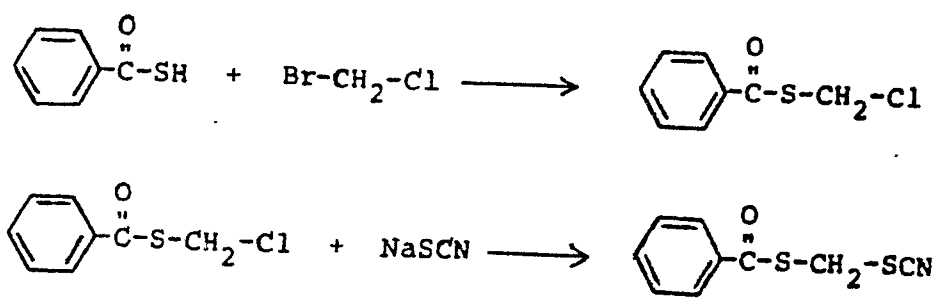

Bevorzugte Dihalogenmethane im Rahmen der Formel (V) sind Verbindungen der Formel

$$X^1—CH_2—X^2 \qquad (IX)$$

worin

$X^1$ und $X^2$ gleich oder verschieden sind und für Fluor, Chlor, Brom oder Jod stehen.

Insbesondere werden die Halogenmethane bevorzugt, die Chlor und/oder Brom enthalten.

Die Herstellung der Dihalogenmethane ist bekannt und kann beispielsweise durch Chlorieren von Methan erfolgen.

Im einzelnen seien die folgenden Dihalogenmethane genannt: Chlor-brom-methan, Dibrommethan, Chlor-jod-methan und Dichlormethan.

Die in der ersten Reaktionsstufe entstehenden Aryl-thiocarbonsäure-halogenmethylester sind bekannt (GB—PS 1 299 134) und können selbstverständlich unter Einsparung der ersten Reaktionsstufe zur Herstellung der Aryl-thiocarbonsäure-thiocyanmethylester eingesetzt werden. Beispielsweise seien die folgenden Aryl-thiocarbonsäure-halogenmethylester genannt:

4-Chlor-thiobenzoesäure-chlormethylester, Thiobenzoesäure-chlormethylester.

Als für die zweite Reaktionsstufe der bevorzugten Ausführungsform des erfindungsgemäßen Verfahrens eingesetzten Thiocyanate seien die Alkali-, Erdalkali- oder Ammoniumthiocyanate genannt, die in molarem Verhältnis oder auch im Überschuß eingesetzt werden können. Beispielsweise seien die folgenden Thiocyanate genannt:

Natriumthiocyanat, Kaliumthiocyanat und Ammoniumthiocyanat.

Die bevorzugt Ausführungsform des erfindungsgemäßen Verfahrens kann im Temperaturbereich von etwa 0 bis etwa 150°C, bevorzugt von 50 bis 110°C, durchgeführt werden.

Es ist möglich die erste und zweite Reaktionsstufe sowohl in einem "Eintopfverfahren" als auch unter Isolierung der Aryl-thiocarbonsäurehalogenmethylester in einem Zweistufenverfahren durchzuführen.

Das erfindungsgemäße Verfahren kann beispielsweise wie folgt durchgeführt werden:

Das Thiobenzoesäure-Kaliumsalz wird in Propanol mit Bromchlormethan bis zur Umsetzung bei 40 bis 60°C gerührt, überschüssiges Bromchlormethan wird abdestilliert, dem Reaktionsgemisch ein leichter Überschuß an Kaliumrhodanid zugesetzt und bis zur Umsetzung, 2 bis 3 Stunden, am Rückfluß erhitzt. Zur Aufarbeitung wird eingeengt, nach Zugabe von Wasser organisch extrahiert, das Endprodukt durch Verdampfen des Lösungsmittels der organischen Phase gewonnen und gegebenenfalls umkristallisiert oder destilliert.

Insbesondere können die erfindungsgemäßen Verbindungen als Wirkstoffe zum Schutz technischer Materialien vor mikrobieller Zersetzung zur Bekämpfung von Molusken und im Pflanzenschutz eingesetzt werden.

Technische Materialien sind z.B. Kühlschmiermittel, Klebstoffe, Streichmassen, Harzleime, Textilhilfsmittel, Kunststoffe, Leder, Dispersionen, Deckfarben und andere wäßrige Anstrichmittel, Putze und andere für mikrobielle Zersetzung anfällige wäßrige Lösungen oder Suspensionen. Die

erfindungsgemäßen Wirkstoffe sind besonders geeignet zur Gebindekonservierung von Dispersionsfarben.

Die erfindungsgemäßen Wirkstoffe wirken stark abtötend oder hemmend auf Mikroorganismen. Als Mikroorganismen seien beispielsweise Bakterien, Pilze, Hefen, Schleimorganismen, Viren und Algen genannt.

Als Bakterien seien beispielsweise genannt: Escherichia coli, Pseudomonas aeruginosa, Pseudomonas fluorescens, Bacillus subtilis, Bacterium vulgare, Bacillus mycoides und Staphylococcus aureus.

Als Pilze und Hefen seien beispielsweise genannt: Penicillium glaucum, Rhizopus nigricans, Aspergillus niger, Torula utilis, Candida crusei und Candida albicans.

Als algen und Schleimorganismen seien beispielsweise gennant: Phaedodactylum tricornutum Bohlin, Euglena gracilis Klebs, Oscillatoria geminata Meneghini, Stichococcus bacillaris Naegili, Aerobacter aerogenes.

Bei Verwendung der erfindungsgemäßen Wirkstoffen zum Schutz technischer Materialien ist die Menge der eingesetzten Wirkstoffe von der Art und dem Vorkommen der Organismen, der Keimzahl in dem Medium abhängig und kann jeweils durch Testreihen leicht ermittelt werden. Im allgemeinen ist es jedoch ausreichend 0,001% bis 0,5% des Wirkstoffes, bezogen auf das Medium, einzusetzen.

Die erfindungsgemäßen Wirkstoffe haben außerdem eine hervorragende Wirkung auf Schnecken, welche als Überträger für menschen- und tierpathogene Trematoden eine Rolle spielen. So sind die Schnecken für die Übertragung von Schistosomen und Fasciola als Zwischenwirte notwendig.

Beispielsweise wird Schistosoma mansoni, Sambon 1907 durch Biomphalaria glabrata und Bolinus truncatus; Schistosoma haematobium, Weinland 1858 durch Bolinus truncatus und Bolinus globosus; Schistosoma japonicum, Katsurada 1904 durch Oncomelania spp.; Fasciola hepatica durch Lynmaea spp. übertragen. Andere durch Schnecken übertragene Trematoden sind Chlonorchis sinensis, Fasciolopsis buski und Paragonimus westermani.

Da die Entwicklung von Trematoden im Zwischenwirt für Parasiten obligatorisch ist, kann eine Bekämpfung beispielsweise der Schistosomiasis und der Pasciolose außer im Endwirt auch dadurch erfolgen, daß der Zwischenwirt beseitigt und so der Entwicklungszyklus von Schistosoma und Fasciola unterbrochen wird. Es kommt nicht zur Ausbildung von Entwicklungsstadien von Trematoden, die Mensch und/oder Tier befallen können.

Die erfindungsgemäßen Schneckenbekämpfungsmittel werden in üblicher Weise angewendet.

Der bei der Schneckenbekämpfung anzuwendende Dosierungsbereich ist in Abhängigkeit von der zu bekämpfenden Schneckenart und sonstigen Gegebenenheiten starke variabel.

Im allgemeinen werden Verbindungen oder Lösungen in einer Konzentration von $10^{-1}$ bis $10^{-7}$ Gewichtsanteilen Wirkstoff eingesetzt werden. Es sind aber auch höhere und niedere Konzentrationen möglich.

Ein weiteres Anwendungsgebiet ist die Bekämpfung von Pflanzenkrankheiten im Pflanzenschutz.

Da die erfindungsgemäßen Wirkstoffe eine starke fungitoxische und bakteriotoxische Wirkung aufweisen und sie Kulturpflanzen in den zur Bekämpfung von Pilzen und Bakterien notwendigen Konzentrationen nicht schädigen, sind sie für den Gebrauch als Pflanzenschutzmittel zur Bekämpfung von Pilz- und Bakterienkrankheiten geeignet.

Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Verbindungen mit bakteriotoxischer Wirkung finden Verwendung gegen phytopathogene Bakterien, z.B. der Gattungen Pseudomonas, Xanthomonas, Erwinia und Corynebacterium.

Die erfindungsgemäßen Wirkstoffe haben ein breites Wirkungsspektrum und können angewandt werden gegen parasitäre Pilze und Bakterien, die oberirdische Pflanzenteile befallen oder die Pflanzen vom Boden her angreifen, sowie gegen samenübertragbare Krankheitserreger.

Eine besonders gute Wirksamkeit entfalten sie gegen parasitäre Pilze auf oberirdischen Pflanzenteilen; so werden z.B. gute Wirkungen erzielt gegen den Erreger des Apfelschorfs (Venturia inaequalis) sowie gegen pilzliche Krankheiten an Getreide, z.B. den Erreger des Getreiderostes (Puccinia recondita).

Als Pflanzenschutzmittel können die erfindungsgemäßen Wirkstoffe zur Saatgutbehandlung und zur Behandlung oberirdischer Pflanzenteile benutzt werden.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozenten, Vorzugsweise zwischen 0,05 und 0,0001%.

Die erfindungsgemäßen Wirkstoffe können in die üblichen Formulierungen übergeführt werden, wie Lösungen, Emulsionen, Suspensionen, Pulver, Pasten und Granulate. Diese werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermittel und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel

kommen im wesentlichen in Frage: Aromaten, wie Xylol, Toluol, Benzol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glykol sowie deren Äther und Ester, Ketone wie Aceton, Methyläthylketon, Methylisobutylketon oder Cyclohexanon, stark polare Lösungsmittel wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Dichlordifluormethan oder Trichlorfluormethan; als feste Trägerstoffe: natürliche Gesteinsmedien, wie Kaoline, Tonerden, Talkum, Kreide, Quarz, Attapulgit und Montmorillonit oder Diatomenerde und synthetische Gesteinsmedien, wie hochdisperse Kieselsäure, Aluminiumoxid und Silikate; als Emulgiermittel: nichtionogene und anionische Emulgatoren, wie Polyoxyäthylen-fettsäureester, Polyoxyäthylen-fettalkohol-äther, z.B. Alkylaryl-polyglykoläther, Alkylsulfonate, Alkylsulfate und Arylsulfonate; als Dispergiermittel: z.B. Sulfitablaugen und Methylcellulose.

Die erfindungsgemäßen Wirkstoffe können in den Formulierungen und in den Mischungen mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Formulierungen enthalten im allgemeinen zwischen 0,1 und 95 Gew.-% Wirkstoff, vorzugsweise von 0,5 bis 90%.

Überraschenderweise haben die erfindungsgemäßen Aryl-thiocarbonsäuren-thiocyanmethylester eine größere Wirksamkeit als die bekannten Alkyl-thiocarbonsäure-thiocyanmethylester (Arch. Pharm. *294,* 475 bis 478 (1961); Arch. Pharm. *300,* 326 bis 329 (1967); JA 7308492).

In den entsprechenden Aufwandmengen und -konzentrationen zeigen die erfindungsgemäßen Stoffe auch eine akarizide Wirkung und eine Wirkung gegen Hygieneschädlinge.

Das Herstellungsverfahren läßt sich in einfacher Weise und mit großen Ausbeuten durchführen.

## Herstellungsbeispiele

### Beispiel 1

*Thiobenzoesäure-thiocyanmethylester*

A Herstellung über Thiobenzoesäure-chlormethylester

49 g (0,35 Mol) Thiobenzoesäure wurden unter Stickstoff bei 0—10° mit 35 g (0,35 Mol) Triäthylamin versetzt. Diese Mischung wurde bei 0 bis 10° zu 91 g (0,7 Mol) Bromchlormethan zugetropft, dann wurde bei Raumtemperatur 16 Stunden weitergerührt, das Lösungsmittel am Rotavapor entfernt, der Rückstand mit Äthylenchlorid/Wasser ausgeschüttelt, die organische Phase abgetrennt und eingeengt. Die verbliebende Flüssigkeit wurde im Vakuum destilliert. Bei 105—115° bei 0,7 bis 2 torr gingen 50 g (77%) Thiobenzoesäure-chlormethylester über.

4,8 g (0,026 Mol) Thiobenzoesäure-chlormethylester und 3,08 g (0,038 Mol) Natriumthiocyanat wurden in 30 ml n-Propanol 2 Stunden am Rückfluß erhitzt. Dann wurde eingeengt, mit Äthylenchlorid/Wasser ausgeschüttelt, die organische Phase abgetrennt und das Lösungsmittel abdestilliert. Es verblieben 5 g eines schwach gelblichen Öls, das nach Animpfen kristallisiert; Fp 45° (das entspricht einer Ausbeute von 93% des theoretischen Umsatzes).

B Herstellung mit Chlormethyl-thiocyanat

28 g (0,16 Mol) Thiobenzoesäure-Kaliumsalz wurden in 550 ml Methanol gelöst und bei −5° 17 g (0,16 Mol) Chlormethyl-thiocyanat zugetropft. Nach langsamem Erwärmen auf Raumtemperatur wurde noch 30 Minuten bei 40° nachgerührt, eingeengt, mit Wasser/Äthylenchlorid ausgeschüttelt, die organische Phase eingeengt und der ölige Rückstand durch mehrstündiges Erhitzen auf 80—100°/0,5 bis 2 Torr von Chlormethyl-thiocyanatresten befreit; Ausbeute 30 g (das entspricht einer Ausbeute von 90% des theoretischen Umsatzes).

### Beispiel 2

*4-Chlor-thiobenzoesäure-thiocyanmethylester*

Zu 31,5 g (0,15 Mol) 4-Chlorthiobenzoesäure-K-salz in 200 ml Methanol wurden bei 0—10° 16 g (0,15 Mol) Chlormethylthiocyanat zugetropft, dann wurde bis zur Umsetzung zunächst auf Raumtemperatur und dann auf 50—60° erwärmt. Die abgekühlte Mischung wurde unter starkem Rühren auf 1 ltr. kaltes Wasser gegossen, der Niederschlag abgesaugt und getrocknet. Hellrosafarbene Kristalle.

Ausbeute: 31 g (das entspricht einer Ausbeute von 85% des theoretischen Umsatzes).

Zur Trennung wurde die unterschiedliche Löslichkeit von Hauptprodukt und Nebenprodukt in Methanol und Ligroin ausgenutzt.

40 g Rohprodukt (2:1) wurden in 250 ml Methanol aufgekocht, abfiltriert, Filtrat in Wasser gegossen, Niederschlag getrocknet: 20 g (Verhältnis 5:1). Nach Umkristallisieren aus 400 ml Ligroin ergaben sich 15 g weiße Kristalle; Fp 80°.

Beispiel 3

*2,5-Dichlor-thiobenzoesäure-thiocyanmethylester*

A Über 2,5-Dichlor-thiobenzosäure-chlormethylester

214 g (1 Mol) 2,5-Dichlor-thiobenzosäure-K-salz wurden in 500 ml Methanol mit 259 g (2 Mol) Bromchlormethan 3 Stunden am Rückfluß gekocht, dann wurde eingeengt, mit Äthylenchlorid/Wasser ausgeschüttelt, die organische Phase eingeengt und im Vakuum destilliert. Die Fraktion von 128—136° bei 1 Torr enthielt den reinen Chlormethylester, der beim Abkühlen kristallisierte. 82 g leicht gelbliche Kristalle; Fp 76° (das entspricht einer Ausbeute von 32% des theoretischen Umsatzes).

56 g (0,22 Mol) des Chlormethylesters und 27 g (0,33 Mol) Natriumthiocyanat wurden in 150 ml Isobutanol 2 Stunden zum Rückfluß erhitzt. Dann wurde eingeengt, mit Äthylenchlorid/Wasser ausgeschüttelt und die organische Phase wieder eingeengt. 53 g eines gelben, erstarrenden Öls, nach Verrühren mit 70 ml Diäthyläther ergaben sich weiße Kristalle; Fp 86° (das entspricht einer Ausbeute von 87% des theoretischen Umsatzes).

B Herstellung mit Chlormethylthiocyanat

14,5 g (0,05 Mol) 2,5-Dichlor-thiobenzoesäure-K-salz und 6,3 g (0,06 Mol) Chlormethylthiocyanat wurden wie in Beispiel 1 B umgesetzt und verarbeitet. Es ergaben sich 18,5 g einer hellbraunen Flüssigkeit, die nach Verrühren mit Äther und 1-tägigem Stehen teilweise kristallisierte. Der Feststoff wurde abgetrennt: 7,5 g weiße Kristalle; Fp 86° (das entspricht einer Ausbeute von 45% des theoretischen Umsatzes).

Beispiel 4

*4-Methyl-thiobenzoesäure-thiocyanmethylester*

20,5 g (0,11 Mol) 4-Methyl-thiobenzoesäure-K-salz und 11,7 g (0,11 Mol) Chlormethylthiocyanat wurden wie in Beispiel 1 B umgesetzt und verarbeitet. Es ergaben sich 22,5 g eines hellen Pulvers, nach Umkristallisieren aus Ligroin 18 g weißer Kristalle; Fp 55° (das entspricht einer Ausbeute von 80% des theoretischen Umsatzes).

Beispiel 5

*2-Chlor-thiobenzoesäure-thiocyanmethylester*

28,5 g (0,14 Mol) 2-Chlor-thiobenzoesäure-K-salz und 15 g (0,14 Mol) Chlormethylthiocyanat wurden wie in Beispiel 1 B umgesetzt und verarbeitet. 26,3 g gelbliches Öl der Dichte 1,36 (das entspricht einer Ausbeute von 77% des theoretischen Umsatzes).

Beispiel 6

*3-Methyl-thiobenzoesäure-thiocyanmethylester*

10 g (0,052 Mol) 3-Methyl-thiobenzoesäure-K-salz und 5,6 g (0,05 Mol) Chlormethylthiocyanat wurden wie in Beispiel 1 B umgesetzt und verarbeitet. 9,9 g ölige Flüssigkeit der Dichte 1,27 (das entspricht einer Ausbeute von 79% des theoretischen Umsatzes).

Beispiel 7

*3-Trifluormethyl-thiobenzoesäure-thiocyanmethylester*

23 g (0,1 Mol) 3-Trifluormethyl-thiobenzoesäure-K-salz und 14,3 g (0,13 Mol) Chlormethylthiocyanat wurden wie in Beispiel 1 B umgesetzt und verarbeitet. 19 g gelbliches Öl der Dichte 1,4 (das entspricht einer Ausbeute von 69% des theoretischen Umsatzes).

Beispiel 8

*2,4-Dichlor-thiobenzoesäure-thiocyanmethylester*

15,8 g (0,064 Mol) 2,4-Dichlor-thiobenzoesäure-K-salz und 6,3 g (0,06 Mol) Chlormethylthiocyanat wurden wie in Beispiel 1 B umgesetzt und verarbeitet. 13,5 g gelbliches Öl der Dichte 1,45 (das entspricht einer Ausbeute von 81% des theoretischen Umsatzes).

Anwendungsbeispiele

Beispiel 9

Die Verbindungen der Tabelle 1 werden jeweils in Konzentrationen von 0,1 bis 100 mg/l in Allens Nährlösung (Arch. Mikrobiol. *17*, 34 bis 53 (1952)), die in 4 l steriles Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1% Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die mikrobistatische Minimal-Konzentration (MMK) oder größere Wirkstoffkonzentrationen aufweisen, sind nach dreiwöchiger Kultur bei Raumtemperatur noch völlig klar, d.h. die in wirkstoffreien Nährlösungen nach 3 bis 4 Tagen bemerkbare starke Vermehrung der Mikroben und Schleimbildung unterbleibt. Aus den in der Tabelle 1 aufgeführten MMK-Werten geht die

antimikrobielle Wirksamkeit der erfindungsgemäßen Wirkstoffe gegenüber Schleimorganismen hervor (Spalte A).

Beispiel 10

Eine Mischkultur von Grün-, Blau-, Braun- und Kieselalgen (Stichococcus bacillaris Naegeli, Euglena gracilis Klebs, Chlorella pyrenoidosa Chick, Phormidium foredarum Gromont, Oscillatoria geminata Meneghini und Phaedodactylum tricornutum Bohlin) wird unter Durchperlen von Luft in Allen's Nährlösung (Arch. Mikrobiol. *17,* 34 bis 53, (1952)), die auf 4 l steriles Wasser, 0,2 g Ammoniumchlorid, 4.0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe der erfindungsgemäßen Wirkstoffe erkennt man an dem Entfärben der Nährlösung. Tabelle 1, Spalte B gibt für die einzelnen Wirkstoffe den MMK-Wert an.

TABELLE I

Spalte A: Testorganismen: Schleimorganismen, die aus Spinnwasser-Kreisläufen bei der Polyamid-Herstellung isoliert werden; Angabe der MMK in mg/l (Beispiel 10)

Spalte B: Testorganismen: Algen-Mischkultur; Angabe der Abtötekonzentration in mg/l (Beispiel 11)

| Wirkstoff | A | B |
|---|---|---|
| $Ar{-}\overset{\overset{O}{\|}}{C}{-}S{-}CH_2{-}SCN$ | | |
| Ar = Phenyl | 5 – 10 | 50 – 100 |
| = 4-Chlor-phenyl | 10 – 20 | 20 – 50 |
| = 2,5-Dichlorphenyl | 10 – 20 | 100 |
| = 4-Methylphenyl | 5 – 20 | 20 – 50 |
| = 2-Chlorphenyl | 5 – 10 | 100 |
| = 3-Methylphenyl | 10 – 20 | 20 – 50 |
| = 3-Trifluormethylphenyl | 10 | 50 – 100 |
| = 2,4-Dichlorphenyl | 10 – 50 | 50 – 100 |

Beispiel 11
(Wirkung gegen Schimmel und Hefen)

Ein Agar, der aus Bierwürze und Pepton hergestellt wird, wird mit den in Tabelle II angegebenen Wirkstoffen in Konzentrationen von 2 mg/l bis 5000 mg/l versetzt. Nach Erstarren des Agars erfolgt die Kontamination mit Reinkulturen von Penicillium glaucum, Chaetomium globosum und Aspergillus niger. Nach zweiwöchiger Lagerung bei 28°C und 60 bis 70% relativer Luftfeuchtigkeit wird die MMK bestimmt. MMK ist die niedrigste Konzentration an Wirkstoff, bei der keinerlei Bewuchs durch die verwendete Mikrobenart erfolgt; sie ist in nachstehender Tabelle II angegeben:

## TABELLE II

Angabe der MMK in mg/l bei der Einwirkung der unten angagebenen Wirkstoffe auf Schimmel und Hefen

| Wirkstoff | Penicillium glaucum MMK (mg/l) | Chaetomium globosum MMK (mg/l) | Aspergillus niger MMK (mg/l) |
|---|---|---|---|
| 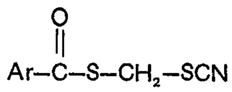 | | | |
| Ar = Phenyl | 5 | 7.5 | 5 |
| = 4-Chlorphenyl | 5 | 3,5 | 3,5 |
| = 2,5-Dichlorphenyl | 20 | 5 | 3,5 |
| = 4-Methylphenyl | 10 | 5 | 7,5 |
| = 2-Chlorphenyl | 10 | 2 | 5 |
| = 3-Methylphenyl | 10 | 5 | 5 |
| = 3-Trifluormethylphenyl | 20 | 20 | 7.5 |
| = 2,4-Dichlorphenyl | 10 | 1,5 | 1 |
| 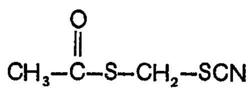 zum Vergleich | 10 | 20 | 20 |

### Beispiel 12
(Wirkung gegen Bakterien)

Ein Agar, der als Nährmedium Bouillon enthält, wird mit den in Tabelle III angegebenen Wirkstoffen in Konzentrationen von 1 bis 5000 ppm versetzt. Darauf infiziert man das Nährmedium jeweils mit Bacterium coli, Bacterium pyocyaneum oder Aerobacter aerogenes und hält das infizierte Medium 2 Wochen bei 28°C und 60 bis 70% relativer Luftfeuchtigkeit. Die MMK ist die niedrigste Konzentration an Wirkstoff, bei der keinerlei Bewuchs durch die verwendete Mikrobenart erfolgt.

Die MMK-Werte sind in Tabelle III wiedergegeben.

## TABELLE III

Angabe der MMK-Werte in mg/l bei der Einwirkung der unten angegebenen Wirkstoffe auf Bakterien.

| Wirkstoff | Bacterium coli MMK (mg/l) | Bacterium pyocyaneum MMK (mg/l) | Aerobacter aerogenes MMK (mg/l) |
|---|---|---|---|
| 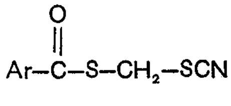 | | | |
| Ar = Phenyl | 350 | 200 | 50 |
| = 2-Chlorphenyl | 200 | 150 | – |
| = 3-Trifluormethylphenyl | 750 | 350 | – |

Beispiel 13

Schneckentest

Als Testschnecken dienen Schnecken der Art Biomphalaria glabratus, die 24 Stunden in einer entsprechenden Verdünnung der zu prüfenden Substanz exponiert werden. Nach weiteren 24 Stunden in wirkstoffreiem Wasser wird das Versuchsergebnis im Vergleich zu unbehandelten Kontrollen ausgewertet.

Das Ergebnis wird in % ausgedrückt. 100% bedeuten, daß alle Schnecken abgetötet, wurden, 0%, daß alle Schnecken überlebten.

TABELLE IV

| Wirkstoff | Wirkstoff-konzentrat. in ppm | abtötende Wirkung in % gegen Biophalaria glabrata |
|---|---|---|
| 2-Chlor-thiobenzoesäure-thiocyanmethylester | 10 | 100 |
| | 3 | 100 |
| | 1 | <50 |
| | 0 | 0 |
| 2,5-Dichlor-thiobenzoesäure-thiocyanmethylester | 10 | 100 |
| | 3 | 0 |
| 3-Chlor-thiobenzoesäure-thiocyanmethylester | 10 | 100 |
| | 3 | 0 |
| 2,4-Dichlor-thiobenzoesäure-thiocyanmethylester | 1 | 100 |
| | 0,3 | – |

Beispiel 14

Die Wirksamkeit von Thiobenzoesäure-thiocyanmethylester gegenüber Meeresalgen (Enteromorpha intestinalis, Ectocarpus spec.) wird wie folgt ermittelt:

Meereswasser, hergestellt aus handelsüblichem Meeressalz und zusätzlich versetzt mit Vitamin V 12 (1 mg/l), $NaNO_3$ (100 mg/l), $Na_2HPO_4$ $12H_2O$ (20 mg/l) und Erddekokt (50 ml/l) Biologie der Algen, Georg Thieme Verlag, Stuttgart, 1968) wird mit den obengenannten Algenspezies kontaminiert. Nach 4 Wochen bei Tageslicht und 18°C werden Proben der Nährlösung in steigender Konzentration (0—100 mg/l) mit Thiobenzoesäure-thiocyanmethylester versetzt.

*Ergebnis:*

10—20 mg/l der erfindungsgemäßen Substanz wirken abtötend auf Ectocarpus spec., 20—40 mg/l abtötend auf Enteromorpha intestinalis.

Beispiel 15

Eine handelsübliche Dispersionsfarbe wird mit 0—0,5% Thiobenzoesäure-thiocyanmethylester versetzt. Die Proben werden wie folgt geprüft:

A. Gebindekonservierung

Während 2 Monaten dreimal Kontamination mit Bakterien (Pseudomonas aeruginosa, Escherichia coli) und Schimmelpilzen (Aspergillus niger, Pullularia pullulans): etwa $10^5$ Keime/g Dispersionsfarbe.

*Ergebnis:*

In Proben, die 0,1% und mehr der erfindungsgemäßen Substanz enthalten, werden die eingebrachten Mikroben jeweils abgetötet; diese Proben sind gut konserviert.

Eine Probe, die nicht mit dem Wirkstoff versetzt ist, enthält am Ende der Prüfzeit mehr als $10^6$ Keime/g.

B. Prüfung von Anstrichen auf Schimmelfestigkeit

Kartonprüfung (5 × 5 cm) werden beidseitig mit Proben eines handelsüblichen Anstrichmittels gestrichen, nach achttägiger Trocknung bei Raumtemperatur in Petrischalen auf einen Traubenzucker-Nährboden gelegt und mit einer Sporensuspension der nachstehenden Pilze kontaminiert (Report 219 der Defense Standards Laboratories Maribuyrnong Australien):

Penicillium citrinum
Stachybotrys atra Corda
Aspergillus flavus Link
Aspergillus ustus
Aspergillus niger
Paecilomyces varioti Bainier
Alternaria tenuis
Cladosphorium herbarum Link et Fries
Pullularia pullulans Fusey

Die kontaminierten Schalen werden bei 28—30°C und 90—95% gelagert und nach 3 Wochen geprüft.

*Ergebnis:*

Anstriche, die bezogen auf den Feststoffgehalt 0,5% und mehr der erfindungsgemäßen Substanz enthalten, sind am Ende der Prüfzeit pilzfrei und gelten als schimmelfest. Wirkstoffreie Anstriche verpilzen unter diesen Bedingungen vollkommen.

## Beispiel 16

Fusicladium-Test (Apfel)/Protektiv

| | |
|---|---|
| Lösungsmittel: | 4,7 Gew.-Tle. Aceton |
| Emulgator: | 0,3 Gew.-Tle. Alkyl-aryl-polyglykoläther |
| Wasser: | 95 Gew.-Tle. |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, wirkstoffkonzentrationen und Ergebnisse gehen aus der Tabelle V hervor:

TABELLE V

Fusicladium-Test (Apfel) / protektiv

| Wirkstoff | Befall in % bei einer Wirkstoffkonzentration von 0,0025 |
|---|---|
| 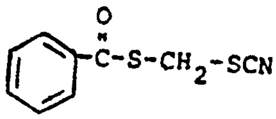 | 40 |
| 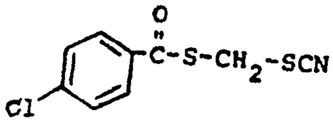 | 0 |
| 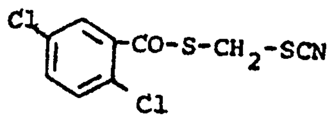 | 1 |
| 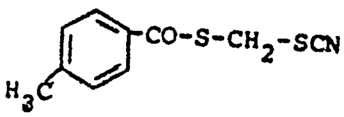 | 0 |
| 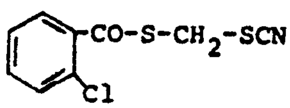 | 6 |
| 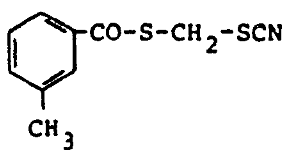 | 70 |
| 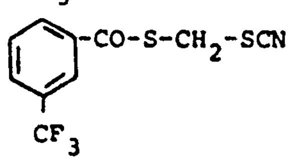 | 26 |
| 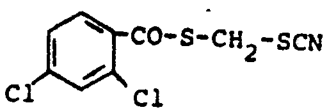 | 11 |
| Vergleich: | |
| $H_3C-CO-S-CH_2-SCN$ | 76 |

### Beispiel 17
### Myzelwachstums-Test

Verwendeter Nährboden:

20 Gew.-Tle. Agar-Agar
200 Gew.-Tle. Kartoffeldekokt
5 Gew.-Tle. Malz
15 Gew.-Tle. Dextrose
5 Gew.-Tle. Pepton
2 Gew.-Tle. $Na_2HPO_4$
0,3 Gew.-Tle. $Ca(NO_3)_2$

Verhältnis von Lösungsmittelgemisch zum Nährboden:

2 Gew.-Tle. Lösungsmittelgemisch
100 Gew.-Tle. Agarnährboden

Zusammensetzung Lösungsmittelgemisch

0,19 Gew.-Tle. Dimethylformamid oder Aceton
0,01 Gew.-Tle. Alkylarylpolyglykoläther
1,80 Gew.-Tle. Wasser

---

2 Gew.-Tle. Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Druchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten und einem Bakterium beimpft und bei etwa 21°C inkubiert.

Die Auswertung erfolgt je nach der Wachstumsgeschwindigkeit der Organismen nach 4—10 Tagen. Bei der Auswertung wird das radiale Wachstum auf den behandelten Nährböden mit dem Wachstum auf dem Kontrollnährboden verglichen. Die Bonitierung des Wachstums geschieht mit folgenden Kennzahlen:

1 kein Wachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Wirkstoffe, Wirkstoffkonzentrationen und Resultate gehen aus der Tabelle VI hervor:

## TABELLE VI

Myzelwachstums-Test

| Wirkstoffe | Wirkstoffkonzentration (ppm) | Fusarium culmorum | Sclerotinia sclerotiorum | Fusarium nivale | Colletotrichum coffeanum | Rhizoctonia solani | Pythium ultimun | Cochliobolus miyabeanus | Botrytis cinerea | Verticillium alboatrum | Pyricularia oryzae | Phialophora cinerescens | Helminthosporium gramineum | Mycosphaerella musicola | Phytophthora cactorum | Pellicularia sasakii | Xanthomonas oryzae |
|---|---|---|---|---|---|---|---|---|---|---|---|---|---|---|---|---|---|
| | | Pilz und Bakterien | | | | | | | | | | | | | | | |
| 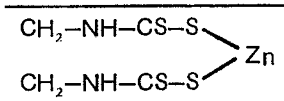 (Vergleich) | 10 | 9 | 9 | 9 | 9 | 9 | 5 | 9 | 9 | 9 | 9 | 9 | 5 | 5 | 9 | 9 | 9 |
| 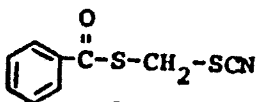 | 10 | 1 | 1 | 1 | 1 | 1 | 1 | 1 | 1 | 1 | 1 | 1 | 1 | 1 | 1 | 1 | 1 |
| 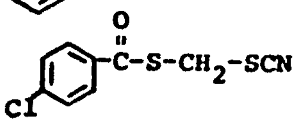 | 10 | 1 | 1 | 1 | 1 | 1 | 1 | 1 | 1 | 1 | 1 | 1 | 1 | 1 | 1 | 1 | 1 |
| 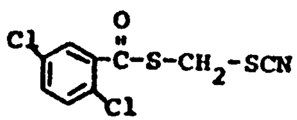 | 10 | 1 | 1 | 1 | 1 | 1 | 1 | 1 | 1 | 1 | 1 | 1 | 1 | 1 | 1 | 1 | 1 |
| 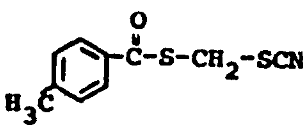 | 10 | 1 | 1 | 1 | 1 | 1 | 1 | 1 | 1 | 1 | 1 | 1 | 1 | 1 | 1 | 1 | 1 |

TABELLE VI

Myzelwachstums-Test

| Wirkstoffe | Wirkstoffkonzentration (ppm) | Fusarium Culmorum | Sclerotinia sclerotiorum | Fusarium nivale | Colletotrichum coffeanum | Rhizoctonia solani | Pythium ultimun | Cochliobolus miyabeanus | Botrytis cinerea | Verticillium alboatrum | Pyricularia oryzae | Phialophora cinerescens | Helminthosporium gramineum | Mycosphaerella musicola | Phytophthora cactorum | Pellicularia sasakii | Xanthomonas oryzae |
|---|---|---|---|---|---|---|---|---|---|---|---|---|---|---|---|---|---|
| | | Pilze und Bakerien | | | | | | | | | | | | | | | |
| 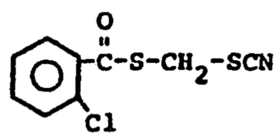 | 10 | 1 | 1 | 1 | 1 | 1 | 1 | 1 | 1 | 1 | 1 | 1 | 1 | 1 | 1 | 1 | 1 |
| 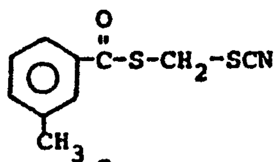 | 10 | 1 | 1 | 1 | 1 | 1 | 1 | 1 | 1 | 5 | 1 | 1 | 1 | 1 | 1 | 1 | 1 |
| 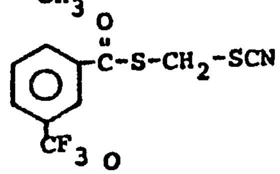 | 10 | 1 | 1 | 1 | 1 | 1 | 1 | 1 | 1 | 1 | 1 | 5 | 1 | 1 | 1 | 1 | 1 |
| 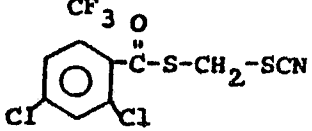 | 10 | 1 | 1 | 1 | 1 | 1 | 1 | 1 | 1 | 1 | 1 | 5 | 1 | 1 | 1 | 1 | 1 |

## Patentansprüche

1. Aryl-thiocarbonsäure-thiocyanmethylester der Formel

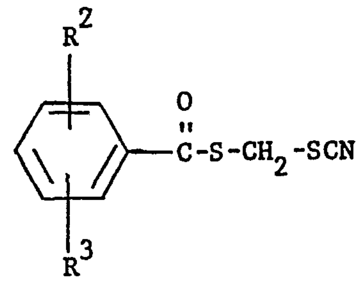

worin

$R^2$ und $R^3$ gleich oder verschieden sind und Wasserstoff, Halogen, $C_1$ bis $C_6$ Alkyl, durch Halogen substituiertes $C_1$ bis $C_6$ Alkyl, $C_1$ bis $C_6$ Alkoxy oder Nitro bedeutet.

2. Verfahren zur Herstellung von Aryl-thiocarbonsäure-thiocyanmethylestern nach Anspruch 1, dadurch gekennzeichnet, daß man Arylthiocarbonsäuren der Formel

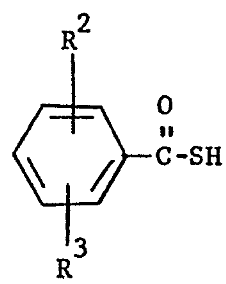

worin

$R^2$ und $R^3$ die obengenannte Bedeutung haben, in Gegenwart eines Säurebinders gegebenenfalls in Gegenwart eines Lösungsmittels mit einer Verbindung der Formel

$$X—CH_2—R^6$$

worin

X für Halogen und

$R^6$ für Halogen oder die Thiocyangruppe steht, und dann gegebenenfalls mit einem Thiocyanat umsetzt.

3. Pestizides Mittel, gekennzeichnet durch einen Gehalt an Aryl-thiocarbonsäure-thiocyanmethylestern nach Anspruch 1.

4. Verwendung von Aryl-thiocarbonsäure-thiocyanmethylestern nach Anspruch 1 zum Schutz technischer Materialien vor mikrobieller Zersetzung.

5. Verwendung von Aryl-thiocarbonsäure-thiocyanmethylestern nach Anspruch 1 zur Bekämpfung von Mollusken.

6. Verwendung von Aryl-thiocarbonsäure-thiocyanmethylestern nach Anspruch 1 zum mikrobiellen Schutz von Pflanzen.

## Revendications

1. Arylthiocarboxylates de thiocyanométhyle, caractérisé en ce qu'ils répondent à la formule générale:

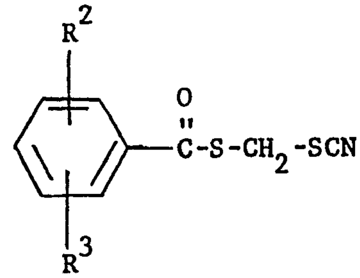

dans laquelle $R^2$ et $R^3$ sont identiques ou différents et représentent chacun un atome d'hydrogène ou d'halogène ou un groupe alkyle en $C_1$—$C_6$, halogénoalkyle en $C_1$—$C_6$, alcoxy en $C_1$—$C_6$ ou nitro.

2. Procédé pour la préparation d'arylthiocarboxylates de thiocyanométhyle selon la revendication 1, caractérisé en ce que l'on fait réagir des acides arylthiocarboxyliques de formule générale:

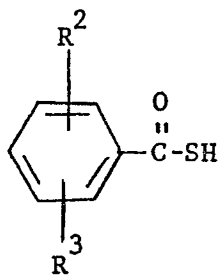

dans laquelle

$R^2$ et $R^3$ sont tels que définis ci-dessus, en présence d'un accepteur d'acide et, éventuellement, en présence d'un solvant, avec un composé de formule générale:

$$X—CH_2—R^6$$

dans laquelle

X représente un halogène et

$R^6$ représente un atome d'halogène ou un groupe thiocyano, et, ensuite, on fait réagir éventuellement avec un thiocyanate.

3. Agent pesticide, caractérisé en ce qu'il contient des arylthiocarboxylates de thiocyanométhyle selon la revendication 1.

4. Utilisation d'arylthiocarboxylates de thiocyanométhyle selon la revendication 1, pour la protection de matériaux techniques contre la dégradation microbienne.

5. Utilisation d'arylthiocarboxylates de thiocyanométhyle selon la revendication 1, pour la lutte contre les mollusques.

6. Utilisation d'arylthiocarboxylates de thiocyanométhyle selon la revendication 1, pour la protection microbienne des plantes.

**Claims**

1. Aryl-thiocarboxylic acid thiocyanomethyl esters of the formula

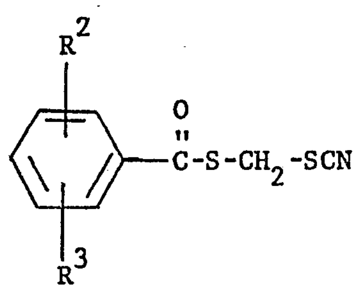

wherein

$R^2$ and $R^3$ are identical or different and denote hydrogen, halogen, $C_1$ to $C_6$ alkyl, $C_1$ to $C_6$ alkyl which is substituted by halogen, $C_1$ to $C_6$ alkoxy or nitro.

2. Process for the preparation of aryl-thiocarboxylic acid thiocyanomethyl esters according to claim 1, characterised in that arylthiocarboxylic acids of the formula

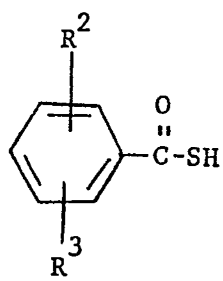

wherein

$R^2$ and $R^3$ have the above-mentioned meaning, are reacted with a compound of the formula

$$X—CH_2—R^6$$

wherein

X stands for halogen and

$R^6$ denotes halogen or the thiocyano group, in the presence of an acid-binding agent and optionally in the presence of a solvent, and then, if appropriate, the products are reacted with a thiocyanate.

3. Pesticidal agent, characterised in that it contains aryl thiocarboxylic thiocyanomethyl esters according to claim 1.

4. Use of aryl thiocarboxylic thiocyanomethyl ester according to claim 1 for protecting industrial materials from microbial decomposition.

5. Use of aryl thiocarboxylic thiocyanomethyl esters according to claim 1 for combating molluscs.

6. use of aryl thiocarboxylic thiocyanomethyl esters according to claim 1 for the microbial protection of plants.